(19) Europäisches Patentamt
European Patent Office
Office européen des brevets

(11) Publication number: 0 244 143 A2

# (12) EUROPEAN PATENT APPLICATION

(21) Application number: 87303483.9

(51) Int. Cl.4: C 07 D 319/06

(22) Date of filing: 21.04.87

(30) Priority: 02.05.86 GB 8610853
01.10.86 GB 8623572

(43) Date of publication of application:
04.11.87 Bulletin 87/45

(84) Designated Contracting States:
AT BE CH DE ES FR GB GR IT LI LU NL SE

(71) Applicant: IMPERIAL CHEMICAL INDUSTRIES PLC
Imperial Chemical House Millbank
London SW1P 3JF (GB)

(72) Inventor: Seebach, Dieter
Orellistrasse 3
CH-8044 Zürich (CH)

Zimmermann, Jurg
Ausserschwand
CH-3715 Adelboden (CH)

(74) Representative: Aufflick, James Neil et al
Imperial Chemical Industries PLC Legal Department:
Patents PO Box 6 Bessemer Road
Welwyn Garden City Herts, AL7 1HD (GB)

## (54) Substituted dioxanones and dioxinones.

(57) Compounds of the formulae:

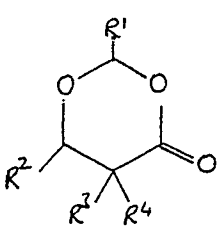

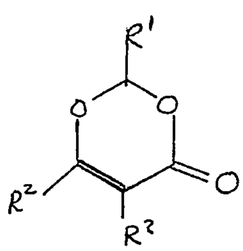

wherein $R^1$, $R^2$, $R^3$ and $R^4$ have a wide range of values are described as useful intermediates in the preparation of pharmaceuticals, etc, in particular in the preparation of optically active β-hydroxy α-substituted carboxylic acids. Processes for their preparation are described.

EP 0 244 143 A2

**Description**

Substituted dioxanones and dioxinones

The present invention relates to chemical compounds and in particular to substituted dioxanones and dioxinones. These compounds are useful intermediates in organic chemistry, for example in preparing substituted β-hydroxycarboxylic acids which have beneficial end uses or themselves are valuable intermediates in organic synthesis such as in the preparation of agrochemicals and pharmaceuticals. This invention further relates to processes of preparing the dioxanones and dioxinones and to their use in preparing β-hydroxycarboxylic acids. It is a particular benefit of this invention that compounds are provided in enantiomerically pure or highly enriched form.

Seebach et al., Angew. Chem. Int. Edition 25 page 178 (1986) and our own copending UK patent application 8525666 describe a process of reacting a compound of the formula (I) with an optically active compound of the formula (II) or a derivative thereof:

$R^aCHO$ (I)

$CH_3CH(OH)CH_2COOH$ (II)

wherein $R^a$ is an organic group, to form a compound of the formula (III):

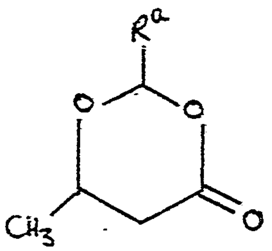

(III)

wherein $R^a$ is as hereinbefore defined.

The compound of the formula (III) is subsequently reacted with a compound of the formula (IV):

$R^b - X$

wherein $R^a$ is an organic group and X is a leaving group, to form an optically active compound of the formula (V) or derivative thereof:

$R^aR^bCH\text{-}O\text{-}CH(CH_3)CH_2COOH$ (V)

wherein $R^a$ and $R^b$ are as hereinbefore defined. The optically active secondary alcohol $R^aR^bCHOH$ is eliminated from the compound of the formula (V) under conventional elimination conditions.

The compound of the formula (II) is used in either R- or S- configuration and is cheap and readily available.

(R)-3-Hydroxybutanoic acid is readily available by the depolymerisation of the microbiologically synthesised poly(R)-3-hydroxybutanoic acid (PHB). For details of such depolymerisation see Seebach et Züger, Helv. Chim. Acta. 65 495 (1982).

(S)-3-Hydroxybutanoic acid is readily available by yeast reduction of acetoacetic ester, see Seebach et al., Organic Syntheses 63, 1 (1984).

The readily available chiral 3-hydroxybutanoic acids have been disclosed as useful, cheap starting materials in a number of synthetic methods, see for example Seidel et Seebach, Tet. Lett. 2209 (1984) and the references contained therein.

We have now found that the compounds of the formula (III) and related compounds are useful intermediates in the preparation of other substituted dioxanones and of substituted dioxinones. In particular they are of use in preparing optically active dioxanones and, subsequently, optically active β-hydroxycarboxylic acids.

Accordingly the present invention provides a compound of the formula (VI):

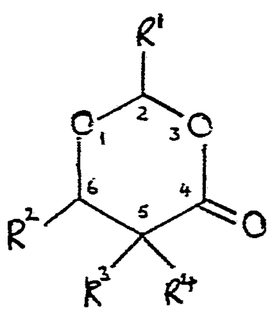

(VI)

wherein:

$R^1$ is an organic group (bonded via a carbon atom),

$R^2$ hydrogen or an organic group (bonded via a carbon atom),

$R^3$ is hydrogen, amino, protected amino, hydroxy, protected hydroxy or an organic group (bonded via a carbon atom), and

$R^4$ is hydrogen, an organic group (bonded via a carbon atom) or is the residue of a heteroelectrophile: with the proviso that $CR^3R^4$ is not $CH_2$, $CHCH_3$ or $C(CH_3)_2$.

In particular, it is preferred that the compounds of the formula (VI) are provided with at least one chiral centre. As will be appreciated the compounds of the formula (VI) have the potential to have chiral centres at the C-2, C-5 and C-6 carbon atoms.

Suitably $R^1$ is $C_{1-10}$ hydrocarbyl, for example $C_{1-10}$alkyl, $C_{2-10}$ alkenyl, $C_{2-10}$ alkynyl, aryl, aryl ($C_{1-4}$) alkyl, $C_{3-10}$ cycloalkyl, $C_{3-7}$ cycloalkyl ($C_{1-4}$) alkyl, heteroaryl, heteroaryl ($C_{1-4}$) alkyl, aryl ($C_{2-4}$) alkenyl, aryl ($C_{2-4}$) alkynyl, heteroaryl ($C_{2-4}$) alkynyl, heteroaryl ($C_{2-4}$) alkenyl, heterocyclyl, heterocyclyl ($C_{1-4}$) alkyl, heterocyclyl ($C_{2-4}$) alkenyl or heterocyclyl ($C_{2-4}$) alkynyl; any of such groups being optionally substituted. Suitably the hetero atoms in the heterocyclyl and heteroaryl moieties are selected from 1 to 4 oxygen, sulphur or nitrogen atoms, the ring systems preferably containing 5 or 6 ring atoms.

Suitable groups for substituting on the group $R^1$ include $C_{1-10}$ alkyl, $C_{2-10}$ alkenyl, $C_{1-10}$ alkylamino, di($C_{1-10}$) alkylamino, amino, hydroxy, $C_{1-10}$ alkoxy, mercapto, $C_{1-10}$alkylthio, halo, nitro, carboxy and the like.

Particular values of $R^1$ are $C_{1-10}$ alkyl and phenyl($C_{1-4}$)alkyl. Preferably $R^1$ is methyl, ethyl, isopropyl, tert-butyl, trichloromethyl, benzyl or phenethyl. In particular $R^1$ is tert-butyl.

Suitably $R^2$ is hydrogen, carboxy or $C_{1-10}$ hydrocarbyl, for example $C_{1-10}$ alkyl, $C_{2-10}$ alkenyl, $C_{2-10}$ alkynyl, aryl, aryl ($C_{1-4}$) alkyl, $C_{3-10}$ cycloalkyl, $C_{3-7}$ cycloalkyl ($C_{1-4}$) alkyl, heteroaryl, heteroaryl, ($C_{1-4}$) alkyl, aryl ($C_{2-4}$) alkenyl, aryl ($C_{2-4}$) alkynyl, heteroaryl ($C_{2-4}$) alkynyl, heteroaryl ($C_{2-4}$) alkenyl, heterocyclyl, heterocyclyl ($C_{1-4}$) alkyl, heterocyclyl ($C_{2-4}$) alkenyl or heterocyclyl ($C_{2-4}$) alkynyl; any of such groups being optionally substituted. Suitably the hetero atoms in the heterocyclyl and heteroaryl moieties are selected from 1 to 4 oxygen, sulphur or nitrogen atoms, the ring systems preferably containing 5 or 6 ring atoms.

Suitable groups for substituting on the group $R^2$ include $C_{1-10}$ alkyl, $C_{2-10}$ alkenyl, $C_{1-10}$ alkylamino, di($C_{1-10}$) alkylamino, amino, hydroxy, $C_{1-10}$ alkoxy, mercapto, $C_{1-10}$alkylthio, halo, nitro, carboxy and the like.

Particular values for $R^2$ are hydrogen, methyl, ethyl, carboxy and trifluoromethyl.

In a most preferred aspect $R^2$ is methyl as this starting material is particularly readily accessible, see formula (II) above.

Suitably $R^3$ is hydrogen, amino, protected amino, hydroxy or protected hydroxy. In one aspect $R^3$ is hydrogen, amino or hydroxy. In particular $R^3$ is hydrogen. Examples of protected amino and protected hydroxy groups are given hereinafter.

In another aspect of $R^3$ is an organic group bonded via a carbon atom.

Suitably $R^4$ is hydrogen or an organic group bonded via a carbon atom.

Examples of Groups from which $R^3$ and $R^4$ may be independently selected include $C_{1-10}$ hydrocarbyl, for example $C_{1-10}$ alkyl, $C_{2-10}$ alkenyl, $C_{2-10}$ alkynyl, aryl, aryl ($C_{1-4}$) alkyl, $C_{3-10}$ cycloalkyl, $C_{3-7}$ cycloalkyl ($C_{1-4}$) alkyl, heteroaryl, heteroaryl ($C_{1-4}$) alkyl, aryl ($C_{2-4}$) alkenyl, aryl ($C_{2-4}$) alkynyl, heteroaryl ($C_{2-4}$) alkynyl, heteroaryl ($C_{2-4}$) alkenyl, heterocyclyl, heterocyclyl ($C_{1-4}$) alkyl, heterocyclyl ($C_{2-4}$) alkenyl or heterocyclyl ($C_{2-4}$) alkynyl; any of such groups being optionally substituted. Suitably the hetero atoms in the heterocyclyl and heteroaryl moieties are selected from 1 to 4 oxygen, sulphur or nitrogen atoms, the ring systems preferably containing 5 or 6 ring atoms.

Suitable groups for substituting on the groups $R^3$ and $R^4$ include $C_{1-10}$ alkyl, $C_{2-10}$ alkenyl, $C_{1-10}$ alkylamino, di($C_{1-10}$) alkylamino, amino, hydroxy, $C_{1-10}$ alkoxy, mercapto, $C_{1-10}$alkylthio, halo, nitro, carboxy and the like.

Preferably $R^3$ is hydrogen or $C_{1-4}$ alkyl for example methyl or ethyl. Preferably $R^4$ is $C_{1-4}$ alkyl for example methyl or ethyl, $C_{2-4}$ alkenyl for example allyl or phenyl ($C_{1-4}$) alkyl for example benzyl or phenethyl.

In another aspect $R^4$ is the residue of a heteroelectrophile. Suitable residues include Br, $C_{1-4}$ alkylthio, optionally substituted phenylthio, optionally substituted phenyl ($C_{1-4}$) alkylthio, $C_{1-4}$ alkylsulphenyl, optionally substituted phenyl ($C_{1-4}$) alkylsulphenyl, $C_{1-4}$ alkylseleno, optionally substituted phenylseleno, optionally substituted phenylselenoxide and $C_{1-4}$ alkylselenoxide. Particular examples are phenylselenyl and phenylselenoxide.

In another aspect the present invention provides a process for preparing the compounds of the formula (VI) which process comprises

(a) reacting a compound of the formula (VII) and a compound of the formula (VIII):

$R^1CHO$ (VII)

$R^2CH(OH)CHR^{31}COOH$ (VIII)

wherein $R^1$ and $R^2$ are as hereinbefore defined and $R^{31}$ is hydrogen, amino, protected amino, hydroxy or protected hydroxy, to form a compound of the formula (VI) wherein $R^3$ is a group $R^{31}$ as hereinabove defined and $R^4$ is hydrogen, and thereafter, if necessary:

(i) when $R^3$ and $R^4$ are both hydrogen, forming an enolate of the formula (IX):

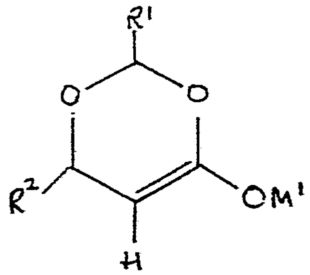

(IX)

wherein $R^1$ and $R^2$ are as hereinbefore defined, and $M^1$ is a counter-ion or protecting group, and subsequently reacting said enolate with an electrophilic source of the group $R^4$ wherein $R^4$ is an organic group to form a compound of the formula (VI) wherein $R^1$ and $R^2$ are as hereinbefore defined, one of $R^3$ and $R^4$ is an organic group and the other is hydrogen; or

(ii) when $R^3$ is a group $R^{31}$ as hereinbefore defined and $R^4$ is hydrogen, forming an enolate of the formula (X):

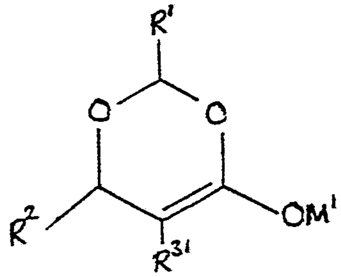

(X)

wherein $R^1$, $R^2$, $R^{31}$ and $M^1$ are as hereinbefore defined, and subsequently reacting said enolate with a heteroelectrophile to form a compound of the formula (VI) wherein $R^1$ and $R^2$ are as hereinbefore defined, $R^3$ is a group $R^{31}$ and $R^4$ is the residue of a heteroelectrophile;

and furthermore, if necessary:

(iii) when $R^4$ is hydrogen and $R^3$ is an organic group in the compound of the formula (VI), reacting said compound to form an enolate of the formula (XI):

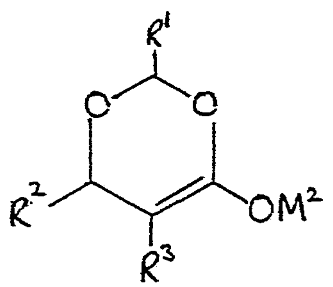

(XI)

wherein $R^1$ and $R^3$ are independently organic groups, $R^2$ is hydrogen or an organic group and $M^2$ is a counter-ion or protecting group, and subsequently either: reacting said enolate with an electrophilic source of the group $R^4$ wherein $R^4$ is an organic group to form a compound of the formula (VI) wherein $R^1$, $R^3$ and $R^4$ are independently organic groups and $R^2$ is hydrogen or an organic group; or reacting said enolate with a heteroelectrophile to form a compound of the formula (VI) wherein $R^1$ and $R^3$ are independently organic groups, $R^2$ is hydrogen or an organic group and $R^4$ is the residue of a heteroelectrophile;

and subsequently, if desired, removing any protecting groups.

It will be appreciated that if an optically active compound the formula (VIII) is used then the products of the present process will also have optical activity.

The reaction, step (a) above, between the compounds of the formulae (VII) and (VIII) provides a compound of the formula (XII)

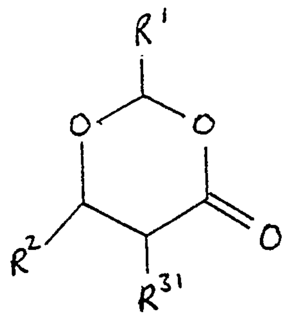

(XII)

This reaction suitably may be performed at ambient or elevated temperature in a solvent. Suitable solvents include halogenated and aromatic hydrocarbons. Preferably for secondary and tertiary aldehydes of the formula (VII) an aromatic hydrocarbon such as benzene or toluene is used. Preferably for primary aldehydes of the formula (VII) chloroform or dichloromethane is used. Conveniently the reaction is performed at reflux with the water formed during the reaction being collected via a Dean-Stark apparatus or the like. The reaction is performed in the presence of a catalyst, for example an acidic catalyst such as sulphuric acid or toluene-4-sulphonic acid.

As mentioned previously, it is a particular benefit of this invention that optically active compounds of the formula (VIII) can be used. The dioxanone products of the formula (XII) are generally produced in a diastereomeric mixture having a ratio of about 9:1. This mixture can be readily purified in conventional manner, but often can be used in the next step of the process of this invention without any ensuing substantial loss of optical purity of the final product.

Particularly useful compounds of the formula (VIII) include whose wherein $R^{31}$ is hydrogen and $R^2$ is methyl, ethyl or trifluoromethyl; wherein $R^{31}$ is hydrogen and $R^2$ is carboxy (malic acid); wherein $R^{31}$ is amino (and alkylated derivatives thereof) and $R^2$ is hydrogen (serine) or methyl (threonine), and wherein $R^{31}$ is hydroxy and $R^2$ is carboxy (tartaric acid). Such compounds are conveniently obtainable in optically active form. In step (i) above, when both $R^3$ and $R^4$ are hydrogen an enolate of the formula (IX) may be formed by the action of a base. The counter-ion $M^1$ is any suitable ion for example an alkali metal ion such as potassium, sodium or lithium. Lithium is preferred. In an alternative $M^1$ is a protecting group such as a silyl moiety for example trimethylsilyl or a magnesium compound for example $M^1$ is a magnesium halide such as - MgBr. Convenient bases for the formation of the enolate are those conventionally used, at low temperatures, for enolate formation in general, for example lithium diethylamide, lithium di-isopropylamide and potassium triphenylmethyl. We have found these anolates to be surprisingly stable. Suitably in the reaction of the compound of the formulae (IX) the electrophilic source of the group $R^4$ is a compound of the formula (XIII):

$R^4 - X^1$ (XIII)

wherein $R^4$ an organic group and $X^1$ is a leaving group for example a silyl group such as trimethylsilyl or a halo atom, for example bromo, iodo and fluoro. Such an alkylating reaction is performed under conventional conditions in a substantially inert solvent such as an ether for example tetrahydrofuran at a low or ambient temperature. In an alternative the source of $R^4$ is a compound that can be introduced by means of an aldol condensation such as an optionally substituted benzaldehyde (PhCHO). Such an aldol condensation is conveniently performed under conventional conditions. Thus step (i) above provides a compound of the formula (XIV):

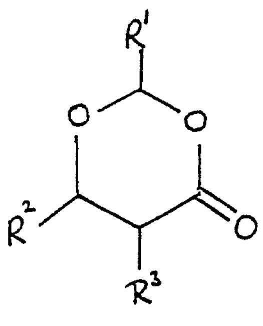

(XIV)

wherein $R^1$ and $R^3$ are independently organic groups ($R^3$ and $R^4$ being interchangeable values) and $R^2$ is hydrogen or an organic group. This alkylation reaction diasteroselectively produces 1,2-trans-disubstituted dioxanones. This is unexpected as 6-membered ring compounds of this substitution pattern normally give 1,2-cis-disubstituted products.

In step (ii) above an enolate compound of the formula (X) is formed, in a manner analogous to that described for enolate (IX), and is reacted with a heteroelectrophile. Suitable heteroelectrophiles include bromine, disulphides, sulphenyl halides and selenyl halides. The reaction between the compound of the formula (X) and the heteroelectrophile can be performed in conventional manner. Conveniently the reaction is performed

under low temperature conditions; in an alternative the heteroelectrophile may be introduced via a radical mechanism.

Thus step (ii) above provides a compound of the formula (XV):

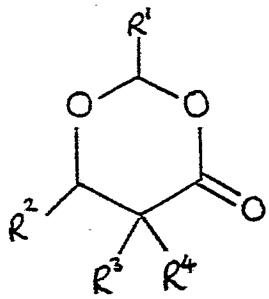

(XV)

wherein $R^1$ is an organic group, $R^2$ is hydrogen or an organic group, $R^3$ is hydrogen, amino, protected amino, hydroxy or protected hydroxy and $R^4$ is the residue of a heteroelectrophile.

In step (iii) described hereinabove a compound of the formula (VI) wherein $R^1$ and $R^3$ are independently organic groups and $R^2$ is hydrogen or an organic group (i.e. a compound of the formula (XIV)) is reacted to form an enolate of the formula (XI) and subsequently reacted with a source of the group $R^4$ to form a compound of the formula (VI) wherein $R^1$, $R^3$ and $R^4$ are all independently organic groups and $R^2$ is hydrogen or an organic group. The formation of the enolate (XI) is analogous to the formation of the enolate (IX) described hereinabove. Values for $M^2$ are as described for $M^1$ above.

In an alternative the compound of the formula (XI) can be reacted with an acid such as acetic acid to reform the compound of the formula (XIV) (i.e. $R^4$ is hydrogen). However the configuration of the C-4 carbon atom can be changed by this procedure.

In a further aspect of step (iii) above, a compound of the formula (VI) wherein $R^3$ is an organic group (i.e. a compound of the formula (XIV)) is reacted to form an enolate of the formula (XI) and then reacted with a heteroelectrophile in a manner analogous to that in which the compounds of the formula (X) are reacted. In this manner compounds of the formula (XVI) are provided:

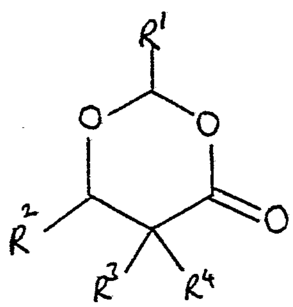

(XVI)

wherein $R^1$ and $R^3$ are independently organic groups, $R^2$ is hydrogen or an organic group and $R^4$ is the residue of a heteroelectrophile.

As stated hereinbefore, the dioxanone compounds of the present invention are of general utility in organic synthesis. In particular they are of special interest when the C-6 and/or C-5 carbon atoms are centres of optical activity. One particularly beneficial use of such compounds is to prepare β-hydroxy α-(mono-or di-)substituted carboxylic acids with a chiral centre at both the α- and β- carbon atoms.

Accordingly in another aspect the present invention provides a process for preparing a compound of the formula (XVII):

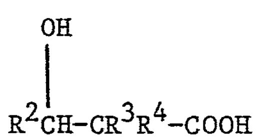

(XVII)

and derivatives thereof, such as protected hydroxy and/or carboxy compounds, wherein $R^2$, $R^3$ and $R^4$ are as hereinbefore defined, which process comprises the acid hydrolysis of a compound of the formula (VI).

Suitable, preferred and particular values for $R^2$, $R^3$ and $R^4$ are as mentioned hereinbefore in relation to dioxanones.

In another aspect of the present invention the compounds of the formula (VI) wherein $R^4$ is a heteroelectrophilic residue can be converted to dioxinones. These are of utility in organic synthesis.

Accordingly the present invention further provides the compounds of the formula (XVIII):

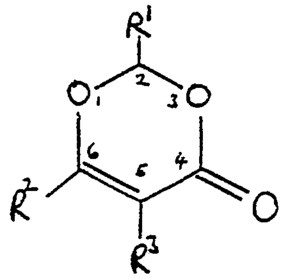

(XVIII)

wherein $R^1$, $R^2$ and $R^3$ are as hereinbefore defined: with the proviso that $R^3$ is not hydrogen or methyl. In a further aspect the present invention provides compounds as depicted in formula (XVIII) in optically active form.

Compounds of the formula (XVIII) are valuable chemical intermediates as they are amenable to a wide variety of chemical reactions, for example nucleophilic attack at the 4-position (acylation) and in the 6-position (Michael addition) and electrophilic attack at the 5-position, and on the $R^2$ group for example after deprotonation if $R^2$ is methyl or ethyl. In addition the carbon-carbon double bond of the compounds of the formula (XVIII) is able to undergo photochemical (2 + 2) cycloadditions. Thus the group $R^2$, the carbon-carbon double bond and the 4-position of the dioxinone ring are capable of undergoing synthetic transformation. Subsequently, or in some cases concurrently, acid-catalysed hydrolysis may be used to remove the $R^1CH$- moiety thus resulting in the target compound, generally an optically active substitued β-hydroxycarboxylic acid. Therefore overall a compound of the formula: $R^2CH(OH)CH_2COOH$ can be submitted at the asymmetric carbon atom without racemisation and without using a chiral auxiliary agent. This is significantly advantageous for practical, commercial syntheses of optically active organic compounds.

Suitable, preferred and particular values for $R^1$, R2 and $R^3$ are as mentioned hereinbefore in relation to dioxanones. In particular $R^2$ is hydrogen, methyl, ethyl, carboxy or trifluoromethyl, especially methyl. In particular $R^1$ is $C_{1-6}$ alkyl or phenyl $C_{1-4}$ alkyl for example isopropyl or tert-butyl. Preferably $R^3$ is hydrogen.

In a further aspect the present invention provides a process for preparing a compound of the formula (XVIII) which comprises eliminating $R^{41}$-H from a compound of the formula (XIX):

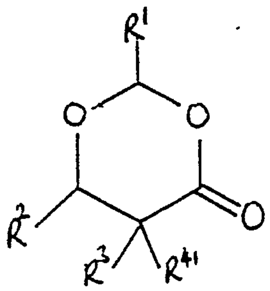

(XIX)

wherein $R^1$, $R^2$ and $R^3$ are as hereinbefore defined and $R^{41}$ is a leaving group.

$R^{41}$ is a leaving group, either being the residue of a heteroelectrophile as described above, or a modification thereof. Elimination of $R^{41}H$ can be effected using base under standard conditions. However, dependent on the nature of $R^{41}$, spontaneous elimination in situ can occur. In an alternative the group $R^{41}$ may be modified to render it liable to spontaneous elimination, for example when $R^{41}$ is phenylselenyl, oxidation to phenylselenoxide causes spontaneous elimination to form the compound of the formula (XVIII). In a particularly preferred embodiment the optically active compounds of the formula (XVIII) wherein $R^3$ is hydrogen may be formed by catalytic hydrogenation of compounds of the formula (XX):

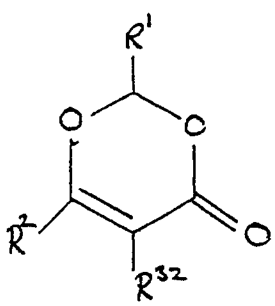

(XX)

wherein $R^1$ and $R^2$ are as hereinbefore defined, and $R^{32}$ is halo, for example bromo, particularly when $R^2$ is methyl. The catalytic hydrogenation is conducted under conventional, mild conditions for example hydrogen gas over palladium on carbon at 1 atmosphere pressure. Case must be taken not to have too vigorous

conditions otherwise the carbon-carbon double bond would reduce. In an alternative the compounds of the formulae (XVIII) wherein $R^3$ is amino or protected amino may be prepared by reacting the compound of the formula (XX) with azide to form the azide, subsequently reducing under mild conditions in conventional manner to form the amino compound and, if desired, forming a protected amino compound for example by acylation. The compounds of formula (XX), which form another aspect of this invention, may be formed by free radical bromination of a compound of the formula (VI) wherein $R^3$ and $R^4$ are both hydrogen, for example using N-bromosuccinimide in the presence of a free radical initiator.

In another aspect the transformation of a compound of the formula (VI) wherein $R^4$ is hydrogen to a compound of the formula (XVIII) may be effected using various dehydrogenation reagents for example utilising a radical mechanism such as anthracene, tetrachloroquinone, tetracyanoquinone, nitrobenzene and heat, selenium and heat.

Protecting groups, for example for forming protected amino and protected hydroxy groups, may in general be chosen from any suitable groups described in the literature and may be introduced in conventional manner. Similarly they may be removed in any convenient and conventional manner, if desired.

Examples of hydroxy protecting groups include $C_{1-4}$alkoxycarbonyl (e.g. t-butoxycarbonyl), aryl$C_{1-4}$ alkoxycarbonyl (e.g. benzyloxycarbonyl), tri($C_{1-4}$)alkylsilyl (e.g. trimethylsilyl) and aryl$C_{1-4}$ alkyl (e.g. benzyl) groups.

Examples of amino protecting groups include aryl$C_{1-4}$alkyl (e.g. benzyl and p-nitrobenzyl), acyl (e.g. t-butoxycarbonyl and benzyloxycarbonyl) silyl (e.g. trimethylsilyl), alkylidene (e.g. methylidene) benzylidene and phthalamido groups.

The following Descriptions and Examples serve to illustrate this invention.

Description 1

A mixture of (R)-3-hydroxybutanoic acid (7.8 g), 3-phenylpropionaldehyde (6.71 g) and pyridinium toluene-4-sulphonic acid (ca. 10 mmol %) in dichloromethane (300 ml) was stirred, under reflux, for 48 hours with removal of water. The reaction mixture was cooled to room temperature, washed three times with saturated sodium bicarbonate, dried ($MgSO_4$) and evaporated under reduced pressure. The residue was crystallised from ether/pentane to give (2R,6R)-2-(2′-phenylethyl)-6-methyl-1,3-dioxan-4-one (7.0 g), m.p. 49.7-50.9°C; $[\alpha]_D^{R.T.} = 15.2$(c = 1.05; $CHCl_3$).

Descriptions 2 - 6

In a manner similar to Description 1 the following dioxanones were obtained in good yield, *3-HB is 3-hydroxybutanoic acid.

| Ex. | Chiral starting-Material | Aldehyde | Dioxanone |
|---|---|---|---|
| 2a | (R)-3-HB | $CH_3(CH_2)_6CHO$ | 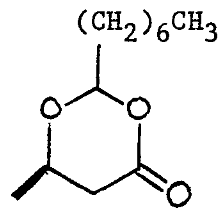 |
| 3a | (R)-3-HB | $(CH_3)_3CCHO$ | 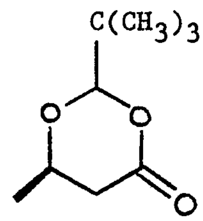 |
| 4a | (R)-3-HB | $(CH_3)_2CHCHO$ | 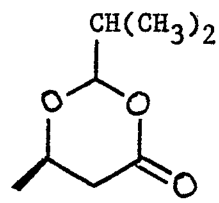 |
| 5a | (R)-3-HB | $Cl_3CCHO$ | 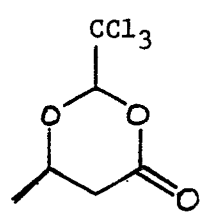 |
| | Chiral starting-Material | Aldehyde | Dioxanone |
| 6a | (S)-3-HB | $C_6H_5CH_2CH_2CHO$ | 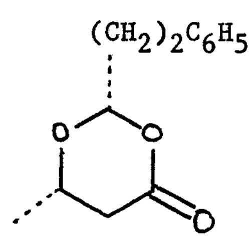 |

Characterising data for Compounds 2a - 6a

(2a) (2R,6R)-2-Heptyl-6-methyl-1,3-dioxan-4-one, Diastereomerically pure, m.pt ca - 20°C, $[\alpha]_D^{R.T.}$ = -33.2 (c=1.85; $CHCl_3$).

IR: ($CHCl_3$) 2970(m); 2940(s); 2870(m); 1740(s); 1460(w); 1390(m); 1350(s); 1290(m); 1260(s); 980(s).

$^1$H-NMR (300 MHz): 0.858 - 0.904 (m, 3H, $CH_3$-$CH_2$); 1.262 -1.480 (m, 10H, 5 $CH_2$); 1.328 (d, J=6.148, 3H, $CH_3$-); 1.739 - 1.81 (m, 2H, $CH_2$-C-O); 2.372 (d x d, $J_1$=17.72, $J_2$-10.68, 1H, HCH); 2.655 (d x d, $J_1$=10.69, $J_2$=4.34, 1H, HC-H); 4.009 (d x d x q, $J_1$= 10.68, $J_2$=4.34, $J_3$=6.148, 1H, Me-CH); 5.295 (t, J=5.014, 1H, O-CH-O).

(3a) (2R,6R)-2-(t-Butyl)-6-methyl-1, 3-dioxan-4-one, Diastereomerically pure, m.pt 82.2-82.8°C, $[\alpha]_D^{R.T.}$ = 56.56° (c=1.28; $CHCl_3$).

(4a) (2R,6R)-2-Isopropyl-6-methyl-1,3-dioxan-4-one, Diastereomeric ratio 96%; colourless oil; $[\alpha]_D^{R.T.}$ = -55.7° (c=1.53; $CHCl_3$).

IR: 2980(m); 2940(w); 2910(w); 2880(w); 1740(s); 1470(w); 1380(m); 1340(m); 1285(m); 1250(s); 1220(m); 1165(m); 985(s); 960(m); 750(m).

$^1$H-NMR (300MHz): 0.995 (d, J=6.88, 3H $CH_3$); 1.004 (d, J=6.87, 3H,$CH_3$); 1.327 (d,J=6.12, 3H,$CH_3$); 1.925 - 2.031 (m, 1H, $CHMe_2$); 2.366 (dxd, $J_1$=17.70, $J_2$-10.67, 1H, H-CH); 2.657 (dxd, $J_1$=17.70, $J_2$=4.36, 1H, HC-H); 3.997 (dxdxq, $J_1$=10.67, $J_2$4.36, $J_3$=6.12, 1H, Me-CH-O); 5.076 (d, J=4.3, 1H, O-CH-O).

(5a) (2R,6R)-6-Methyl-2-trichloromethyl-1,3-dioxan-4-one Diastereomerically pure white crystals, m.pt 113.6 - 114.4°C; $[\alpha]_D^{RT}$ = -54.7° (c=1.62; $CHCl_3$).

(6a) (2S,6S)-2-(2′-Phenylethyl)-6-methyl-1,3-dioxan-4-one Diastereomerically pure white crystals, m.pt 49.5 - 50.4°C; $[\alpha]_D^{RT}$ = 15.4° (c=0.47; $CHCl_3$).

EXAMPLE 1

To di-isopropylamine (2 ml) in tetrahydrofuran (20 ml) was added butyllithium (1.55N in hexane; 8 ml) at 0°C. The solution was cooled to -78°C after 30 minutes. (2R, 6R)-2-(t-Butyl)-6-methyl-1,3-dioxan-4-one (2 g) in tetrahydrofuran (20 ml) was added over 5 minutes to give the corresponding enolate. The solution was stirred for 30 minutes, iodomethane (3.2 ml) added, and the solution was stirred for a further 16 hours at -78°C. The reaction mixture was quenched with pH7 buffer (20 ml), extracted into ether (3 x 20 ml), dried and evaporated under reduced pressure to give (2R, 5R, 6R)-5,6-dimethyl-2-t-butyl-1.3-dioxan-4-one (2.05 g), m.p. 65.5 - 67°C; $[\alpha]_D^{20}$ = 24.05° (c =1.16; $CHCl_3$).

EXAMPLES 2 - 4

In a similar manner the intermediate enolate was alkylated in situ with a variety of electrophiles. The results are given in Table 1.

Table 1

| Ex. | Enolate | Electrophile | Product | Yield % | Diastereo-selectivity |
|---|---|---|---|---|---|
| 1 | 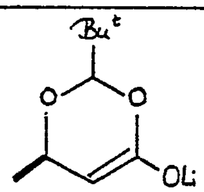 | $CH_3I$ | 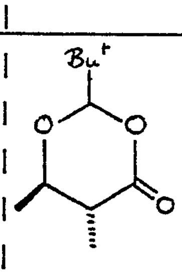 | 94 | >95 |
| 2 | 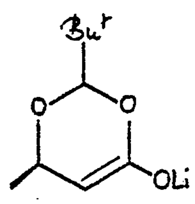 | $C_6H_5CH_2Br$ | 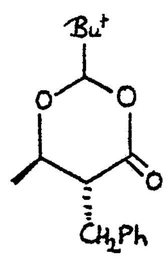 | 80 | >95 |
| 3 | 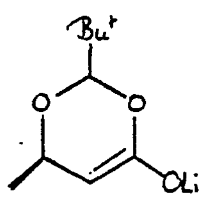 | $CH_2=CHCH_2Br$ | 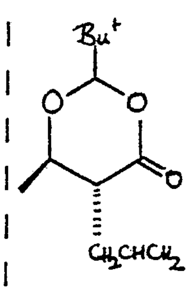 | 85 | >95 |
| 4 | 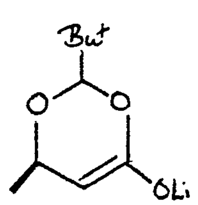 | PhCHO | 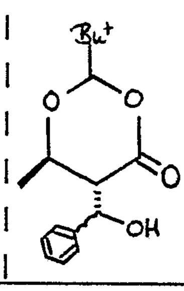 | | 2:1 two isomers epimeric at carbinol. |

EXAMPLES 5 - 8

The product of Example 1 was treated with lithium diisopropylamide in tetrahydrofuran at -75°C to form an enolate. This was alkylated in situ with methyl iodide. The product of Example 2 was treated in analogous manner.

In a similar manner the silylated enolate 7 and the magnesium bromide enolate 8 were formed and reacted. The results are given in Table 2.

Table 2

| Ex. | Enolate | Electrophile | Product | Yield (%) | Diastereo-selectivity (%) |
|---|---|---|---|---|---|
| 5 | 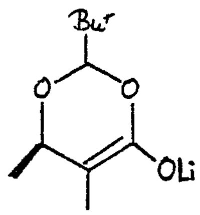 | $CH_3I$ | 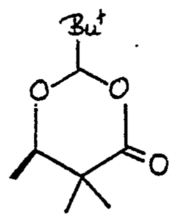 | 90 | - |
| 6 | 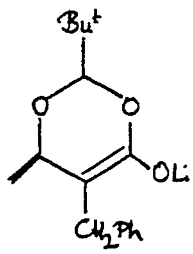 | $CH_3I$ | 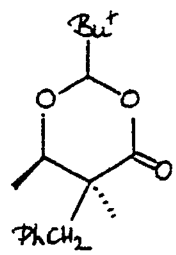 | 60 | >95 |
| 7 | 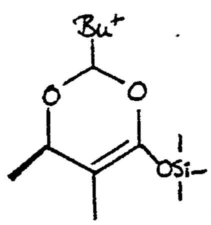 | $NH_4F/H_2O$ | 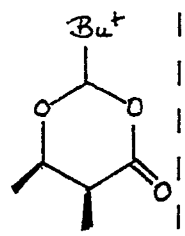 | 90 | 84 |
| 8 | 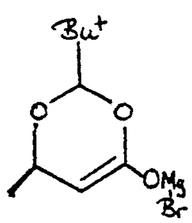 | PhCHO | 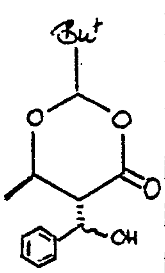 | | 1:2 two isomers epimeric at carbinol. |

EXAMPLE 9

To the enolate of Example 1 (formed from dioxanone (o.5 g) was added phenylselenyl chloride (0.62 g) in tetrahydrofuran (5 ml). The dark solution was stirred for 2 hours, quenched with pH 7 buffer (15 ml), extracted into ether (3 x 20 ml), dried and evaporated under reduced pressure. The crude product was subjected to flash column chromatography (hexane:ether (1:1)) to afford 2-t-butyl-5-phenylselenyl-6-methyl-1,3-dioxan-4-one (0.807 g). This was dissolved in dichloromethane (20 ml); hydrogen peroxide (1 ml; 30% in water) and pyridine (0.5 ml) were added and the mixture was stirred for 2 hours at 20°C. Water (5 ml) was added, the solution extracted into dichloromethane (3 x 20 ml), dried and evaporated under reduced pressure to give, after recrystallisation from ethane/hexane,

(2R)-6-methyl-2-t-butyl-1,3-dioxin-4-one (0.3 g), m.p. 47-49°C;

$[\alpha]_D^{20} = -217.7°$ (c = 1.005; $CHCl_3$).

This compound is reacted with various reagents to demonstrate its versatility as shown in Examples 10 and 11 and Table 3.

EXAMPLE 10

To a solution of di-isopropylamine (0.1 ml) in tetrahydrofuran (2 ml) was added butyllithium (1.55N in hexane; (0.4 ml) at 0°C. The solution was cooled to -78°C andhexamethylphosphoramide (HMPA) (1 equivalent) was added. After 30 minutes, the product of Example 9 (1 equivalent) in tetrahydrofuran (2 ml) was added dropwise to form the corresponding enolate. The solution was stirred for 30 minutes and iodomethane (0.1 ml) was added. The reaction mixture was stirred for 16 hours, the temperature rising to -20°C. The solution was quenched with pH 7 buffer (10 ml), extracted into hexane (3 x 10 ml), dried and evaporated to give an isomeric mixture. γ-Isomer 'H NMR (300 MHz):

1.1(S, 9H, $Bu^t$); 1.15(t, 3H, $CH_3$); 2.3(q,2H,$CH_2$); 5.05 (s, 1H, O-CH-O), 5.38 (s, 1H, =CH). α + γ -Isomer 'H NMR (300 MHz): 1.05 (s, 9H, $Bu^t$); 1.2(t, 3H, $CH_3$); 1.95(s, 3H, =$CCH_3$), 2.4 (m, 2H, $CH_2$); 4.95(s, 1H, O-CH-O).

EXAMPLE 11

To a solution of copper iodide (0.133 g) in ether (5 ml) was added butyl lithium (0.93 ml; 1.55N in hexane) at 0°C. The solution was cooled to -78°C and the product of Example 9 (0.1 g) added. After 100 minutes stirring at -78°C the reaction mixture was quenched with pH 7 buffer (10 ml), concentrated ammonia (5 ml) was added, the solution was extracted with ether ( 3 x 20 ml), dried and evaporated to give (2R)-6-methyl-6-n-butyl-2-t-butyl-1,3-dioxan-4-one (0.11 g) as a yellow oil; 'H NMR (300 MHz): 0.9 (m, 12H, $Bu^t$and $CH_3$); 1.25 (s, 3H, $CH_3$); 1.32 (m, 4H); 1.58 (m, 2H); 2.58 (d x d, J = 15.8 Hz, 2H, $CH_2C$-O); 4.93 (s, 1H, O-CH-O).

Table 3

| Reagent | Product | Yield (%) | Diastereoselectivity (%) |
|---|---|---|---|
| $CH_3I/LiN(C_3H_7-i)_2$ | 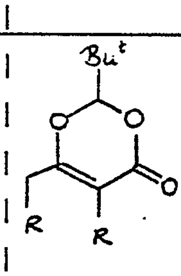 | 80 | 87% γ<br>13% γ + α |
| $Bu^n{}_2CuLi$ | 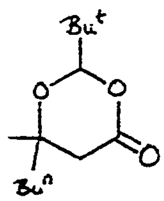 | 82 | >95 |
| cyclohexene/hv | 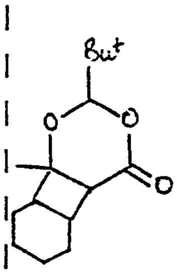 | 90 | mixture |

Table 3 cont'd

| Reagent | Product | Yield (%) | Diastereoselectivity (%) |
|---|---|---|---|
| LDA/$_nC_5H_{11}I$ | 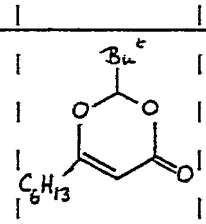 | | |
| N-Bromosuccinimide | 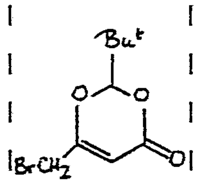 | | |
| $C_2Cl_6/LiN(C_3H_7\text{-}i)_2$ | 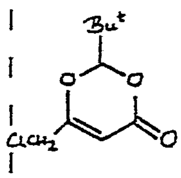 | | |

The products from Tables 1 - 3 are capable of being hydrolysed by acid to form the corresponding substituted β-hydroxycarboxylic acids:

e.g.

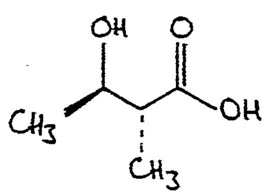

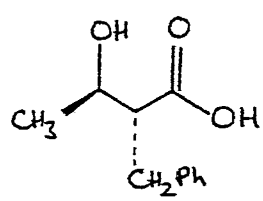

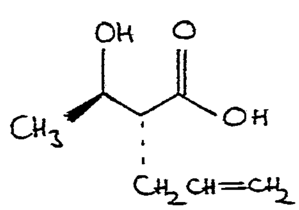

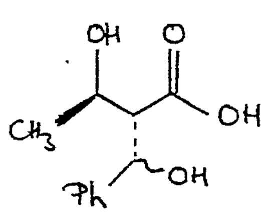

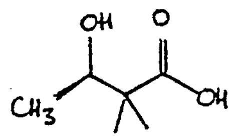

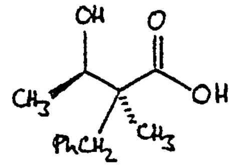

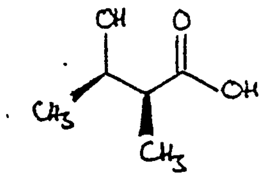

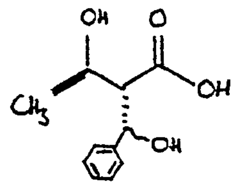

EXAMPLE 12

(2R, 3R)-2-Benzyl-3-hydroxybutyric acid

The product of Example 2 (1.0 g) was dissolved in ethanol (25 ml). Dowex 50W resin (0.2 g) was added and the reaction mixture was stirred for 17 hours at 20° C. The mixture was filtered and evaporated to give the title compound (0.72 g; 97%) m.p. 123-124°C; $[\alpha]_D^{20} = +30.3$ (c = 1.09, ethanol).

EXAMPLE 13

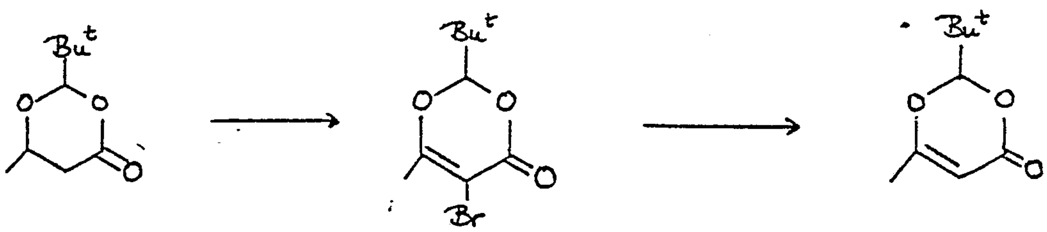

(2R, 6R)-2-(t-Butyl)-6-methyl-1,3-dioxan-4-one (50 g) was dissolved in carbon tetrachloride (1000 ml). N-Bromosuccinimide (78 g) and azoisobutyronitrile (0.5 g) were added and the suspension was heated under reflux until the N-bromosuccinimide had been consumed. The suspension was cooled to 0° C, filtered and evaporated under reduced pressure to give an orange oil (70 g). This oil comprised the bromo intermediate depicted above and was purified by chromatography. $[\alpha]_D^{25} = -183.9°$ (c - 1.17).*

This oil was dissolved in ethanol (1000 ml) and triethylamine (51 ml) and palladium on charcoal (14 g; 10%) were added. The suspension was stirred under an atmosphere of hydrogen until thin layer chromatography (hexane:ether 1:1) showed an absence of bromo intermediate. The catalyst was removed by filtration and washed with dichloromethane. The combined filtrate was evaporated under reduced pressure to give the product. (2R)-6-methyl-2-t-butyl-1,3-dioxin-4-one (30 g) which was purified by flash chromatography (hexane:ether 1:1) to give material identical to that of Example 9.

The above reaction of the dioxanone and N-bromosuccinimide can also givethe 5-bromo-6-bromoethyl by-product. This can also be hydrogenated to provide the dioxinone product and furthermore can be attacked by nucleophiles (e.g. azide) to form 5-bromo-6-substituted-methyl dioxinones (e.g. 5-bromo-6-azidomethyl dioxinones).

* On standing at room temperature for several days a sample of this oil racemised and crystallised (m.p. 85.5-86.5° C).

EXAMPLE 14

In a manner analogous to Example 13, (2R, 6R)-2,6-dimethyl-1,3-dioxan-4-one gave (2R)-5-bromo-2,6-dimethyl,1,3-dioxin-4-one as an oil, purified by flash chromatography, $[\alpha]_D^{25} = -185.9°$ (c = 2.52).

EXAMPLE 15

(3R)-3-Hydroxyvaleric acid (10 g), pivaldehyde (20 ml) and Dowex 50 W resin (1 g) in dichloromethane (120 ml) were stirred under reflux for 17 hours using an inverse water trap. The reaction mixture was filtered, extracted with sodium carbonate and evaporated to give (2R, 6R)-6-ethyl-2-(tert)butyl-1,3-dioxan-4-one,

purified by fractional distillation to give cis-product, b.p. 55°C/0.08 torr; $[\alpha]_D^{25}$ = -37.2° (c = 1.28).

The 6-ethyl compound (1 g), N-bromosuccinimide (2 g) and azobisisobutyronitrile (0.1 g) in carbon tetrachloride were stirred under reflux for 90 minutes. The mixture was cooled to 0°C, filtered and evaporated to give an oil which was purified by flash chromatography (hexane:ether) to give (2R)-6-($1^1$-bromoethyl) -2-(tert)butyl-1,3-dioxin-4-one as an oily mixture of diastereoisomers; $[\alpha]_D^{25}$ - -98.1(c = 2.6). Repeat of this bromination with about 5 equivalents of N-bromosuccinimide gave the di-bromo compound, (2R)-6-(1,$1^1$-di-bromoethyl)-2-(tert)butyl-1,3-dioxin-4-one, m.p. 61°C. $[\alpha]_D^{25}$ = -92.9°(c = 0.86).

The mono-bromo and di-bromo compounds as a crude reaction mixture were dissolved in ethyl acetate and hydrogenated at 1 atmosphere over 10% palladium on carbon for about 6 hours. Purification of the reaction mixture gave (2R)-2-(tert)butyl-6-ethyl -1,3-dioxin-4-one as an oil; $[\alpha]_D^{25}$ = -170.8° (c = 1.39).

EXAMPLE 16

The product of Example 14 (5 g), acetic acid (0.2 ml) and sodium azide (6.5 g) in dimethylformamide (100 ml) were stirred at 25°C for 3 days. Water (400 ml) was added, the mixture was extracted with hexane (3 x 150 ml), dried, evaporated and purified by column chromatography (hexane:ether) to give (2R)-5-azido-2-(tert)butyl-6-methyldioxin-4-one (2.2 g) as an oil, $[\alpha]_D^{25}$ = -218.6° (c = 1.34).

The azide (0.2 g) in ethyl acetate (10 ml) was hydrogenated (1 atmosphere) over 10% palladium on carbon (0.05 g) to give the relatively unstable amine which was characterised by conversion to the corresponding acetamido derivative. The hydrogenation can also be achieved in acetic acid/acetic anhydride to afford the acetamido derivative directly.

**Claims**

1. A compound of the formula (VI):

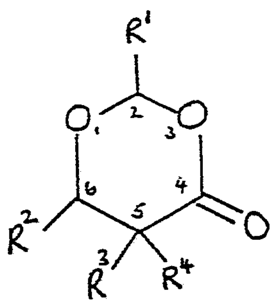

(VI)

wherein:
$R^1$ is an organic group (bonded via a carbon atom),
$R^2$ is hydrogen or an organic group (bonded via a carbon atom),
$R^3$ is hydrogen, amino, protected amino, hydroxy, protected hydroxy or an organic group (bonded via a carbon atom) and
$R^4$ is hydrogen, an organic group (bonded via a carbon atom) or is the residue of a heteroelectrophile: with the proviso that $CR^3R^4$ is not $CH_2$, $CHCH_3$ or $C(CH_3)_2$.

2. A compound of the formula (VI):

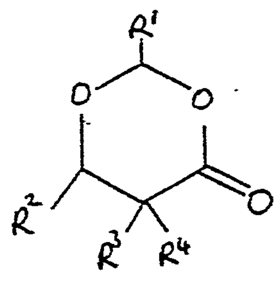

(VI)

in optically active form wherein:
$R^1$ is an organic group (bonded via a carbon atom),
$R^2$ is hydrogen or an organic group (bonded via a carbon atom),
$R^3$ is hydrogen, amino, protected amino, hydroxy, protected hydroxy or an organic group (bonded via a carbon atom), and
$R^4$ is hydrogen, an organic group (bonded via a carbon atom) or is the residue of a heteroelectrophile:

3. A compound according to either claim 1 or claim 2 wherein $R^2$ is hydrogen, methyl, ethyl, carboxy or trifluroromethyl.

4. A compound according to any one of claims 1 to 3 wherein $R^3$ is hydrogen.

5. A compound according to any one of claims 1 to 4 wherein $R^4$ is $C_{1-4}$ alkyl, $C_{2-4}$ alkenyl or phenyl$C_{1-4}$ alkyl.

6. A process for preparing the compounds of the formula (VI) as defined in claim 1 which process comprises

(a) reacting a compound of the formula (VII) and a compound of the formula (VIII):

$R^1CHO$ (VII)

$R^2CH(OH)CHR^{31}COOH$ (VIII)

wherein $R^1$ and $R^2$ are as defined in claim 1 and $R^{31}$ is hydrogen, amino, protected amino, hydroxy or protected hydroxy, to form a compound of the formula (VI) wherein $R^3$ is a group $R^{31}$ as hereinabove defined and $R^4$ is hydrogen, and thereafter, if necessary:

(i) when $R^3$ and $R^4$ are both hydrogen, forming an enolate of the formula (IX):

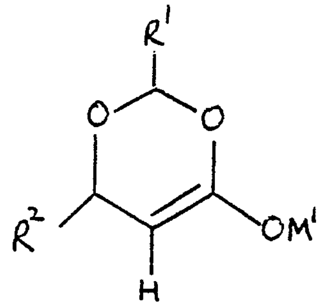

wherein $R^1$ and $R^2$ are as hereinbefore defined, and $M^1$ is a counter-ion or protecting group, and subsequently reacting said enolate with an electrophilic source of the group $R^4$ wherein $R^4$ is an organic group to form a compound of the formula (VI) wherein $R^1$ and $R^2$ are as hereinbefore defined, one of $R^3$ and $R^4$ is an organic group and the other is hydrogen; or

(ii) when $R^3$ is a group $R^{31}$ as hereinbefore defined and $R^4$ is hydrogen, forming an enolate of the formula (X):

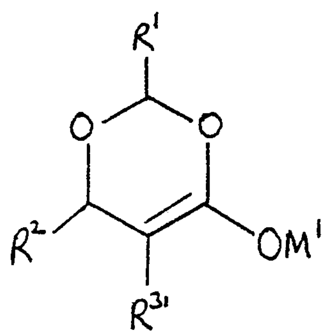

wherein $R^1$, $R^2$, $R^{31}$ and $M^1$ are as hereinbefore defined, and subsequently reacting said enolate with a heteroelectrophile to form a compound of the formula (VI) wherein $R^1$ and $R^2$ are as hereinbefore defined, $R^3$ is a group $R^{31}$ and $R^4$ is the residue of a heteroelectrophile;

and furthermore, if necessary:

(iii) when $R^4$ is hydrogen and $R^3$ is an organic group in the compound of the formula (VI), reacting said compound to form an enolate of the formula (XI):

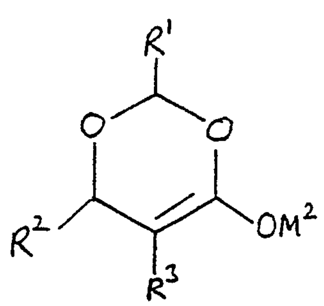

wherein $R^1$ and $R^3$ are independently organic groups, $R^2$ is hydrogen and an organic group and $M^2$ is a counter-ion or protecting group, and subsequently either: reacting said enolate with an electrophilic source of the group $R^4$ wherein $R^4$ is an organic group to form a compound of the formula (VI) wherein $R^1$,

$R^3$ and $R^4$ are independently organic groups and $R^2$ is hydrogen or an organic group; or reacting said enolate with a heteroelectrophile to form a compound of the formula (VI) wherein $R^1$ and $R^3$ are independently organic groups, $R^2$ is hydrogen or an organic group and $R^4$ is the residue of a heteroelectrophile;
and subsequently, if desired, removing any protecting groups.

7. A process for preparing a compound of the formula (XVII):

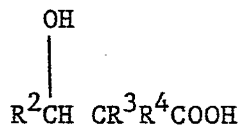

(XVII)

and derivatives thereof, wherein $R^2$,$R^3$ and $R^4$ are as defined in claim 1, which comprises the acid hydrolysis of a compound of the formula (VI).

8. A compound of the formula (XVIII):

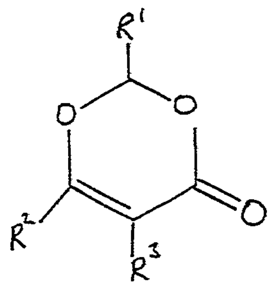

(XVIII)

wherein $R^1$, $R^2$ and $R^3$ are as defined in claim 1 with the proviso that $R^3$ is not hydrogen or methyl.

9. A compound of the formula (XVIII):

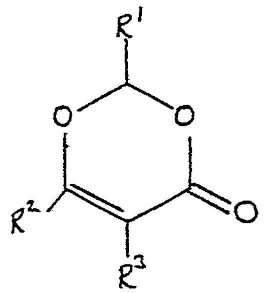

(XVIII)

in optically active form wherein $R^1$, $R^2$ and $R^3$ are as defined in claim 1.

10. A process for preparing a compound of the formula (XVIII) as defined in claim 8 which comprises

(i) eliminating the elements $R^{41}$ and hydrogen from a compound of the formula (XIX):

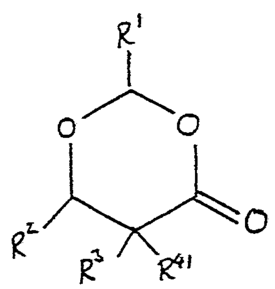

(XIX)

wherein $R^1$, $R^2$ and $R^3$ are as defined in claim 1 and $R^{41}$ is a leaving group; or

(ii) dehydrogenating a compound of the formula (VI), as defined in claim 1, wherein $R^4$ is hydrogen; or

(iii) to form a compound of the formula (XVIII) wherein $R^3$ is hydrogen, catalytically hydrogenating a compound of the formula (XX).

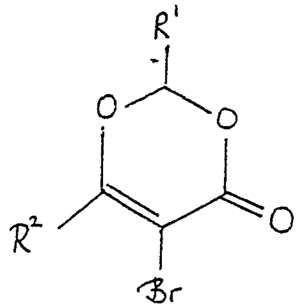

(XX)

wherein $R^1$ and $R^2$ are as hereinbefore defined.

11. A compound of the formula (XX)

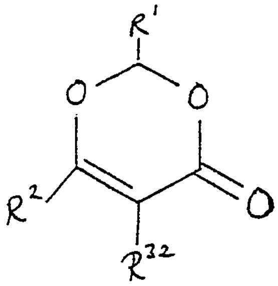

(XX)

wherein $R^1$ and $R^2$ are as defined in claim 1 and $R^{32}$ is halo.

Claims for the following contracting states: AT, ES

1. A process for preparing a compound of the formula (VI):

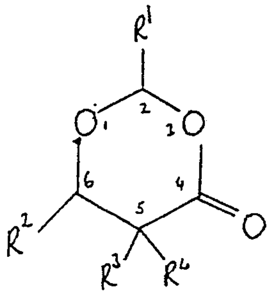

wherein:
$R^1$ is an organic group (bonded via a carbon atom),
$R^2$ is hydrogen or an organic group (bonded via a carbon atom),
$R^3$ is hydrogen, amino, protected amino, hydroxy, protected hydroxy or an organic group (bonded via a carbon atom), and
$R^4$ is hydrogen, an organic group (bonded via a carbon atom) or is the residue of a heteroelectrophile: with the proviso that $CR^3R^4$ is not $CH_2$, $CHCH_3$ or $C(CH_3)_2$: which process comprises
(a) reacting a compound of the formula (VII) and a compound of the formula (VIII):
$R^1CHO$ (VII)
$R^2CH(OH)CHR^{31}COOH$ (VIII)
wherein $R^1$ and $R^2$ are as defined hereinbefore and $R^{31}$ is hydrogen, amino, protected amino, hydroxy or protected hydroxy, to form a compound of the formula (VI) wherein $R^3$ is a group $R^{31}$ as hereinabove defined and $R^4$ is hydrogen, and thereafter, if necessary:
(i) when $R^3$ and $R^4$ are both hydrogen, forming an enolate of the formula (IX):

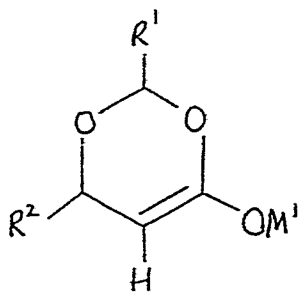

wherein $R^1$ and $R^2$ are as hereinbefore defined, and $M^1$ is a counter-ion or protecting group, and subsequently reacting said enolate with an electrophilic source of the group $R^4$ wherein $R^4$ is an organic group to form a compound of the formula (VI) wherein $R^1$ and $R^2$ are as hereinbefore defined, one of $R^3$ and $R^4$ is an organic group and the other is hydrogen; or

(ii) when $R^3$ is a group $R^{31}$ as hereinbefore defined and $R^4$ is hydrogen, forming an enolate of the formula (X):

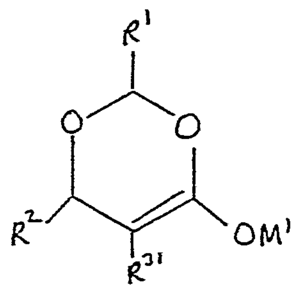

wherein $R^1$, $R^2$, $R^{31}$ and $M^1$ are as hereinbefore defined, and subsequently reacting said enolate with a heteroelectrophile to form a compound of the formula (VI) wherein $R^1$ and $R^2$ are as hereinbefore defined, $R^3$ is a group $R^{31}$ and $R^4$ is the residue of a heteroelectrophile;

and furthermore, is necessary:

(iii) when $R^4$ is hydrogen and $R^3$ is an organic group in the compound of the formula (VI), reacting said compound to form an enolate of the formula (XI):

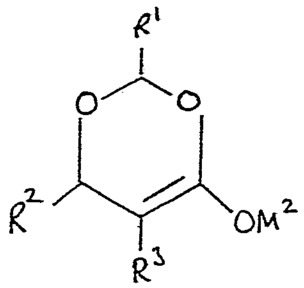

wherein $R^1$ and $R^3$ are independently organic groups, $R^2$ is hydrogen or an organic group and $M^2$ is a counter-ion or protecting group, and subsequently either: reacting said enolate with an electrophilic source of the group $R^4$ wherein $R^4$ is an organic group to form a compound of the formula (VI) wherein $R^1$, $R^3$ and $R^4$ are independently organic groups and $R^2$ is hydrogen or an organic group; or reacting said enolate with a heteroelectrophile to form a compound of the formula (VI) wherein $R^1$ and $R^3$ are independently organic groups, $R^2$ is hydrogen or an organic group and $R^4$ is the residue of a heteroelectrophile;

and subsequently, if desired, removing any protecting groups.

2. A process as depicted in claim 1 for preparing a compound of the formula (VI):

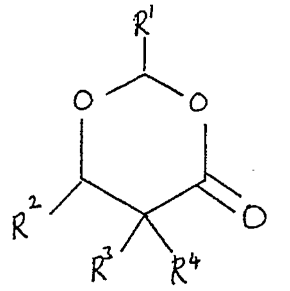

(VI)

in optically active form wherein:
$R^1$ is an organic group (bonded via a carbon atom),
$R^2$ is hydrogen or an organic group (bonded via a carbon atom),
$R^3$ is hydrogen, amino, protected amino, hydroxy, protected hydroxy or an organic group (bonded via a carbon atom), and
$R^4$ is hydrogen, an organic group (bonded via a carbon atom) or is the residue of a heteroelectrophile.

3. A process according to either claim 1 or claim 2 for preparing a compound wherein $R^2$ is hydrogen, methyl, ethyl, carboxy or trifluoromethyl.

4. A process according to any one of claims 1 to 3 for preparing a compound wherein $R^3$ is hydrogen.

5. A process according to any one of claims 1 to 4 for preparing a compound wherein $R^4$ is $C_{1-4}$ alkyl, $C_{2-4}$ alkenyl or phenyl$C_{1-4}$ alkyl.

6. A process for preparing a compound of the formula (XVII):

EMI PA=28 FR=2 HE=10 WI=100 TI=CHE

and derivatives thereof, wherein $R^2$,$R^3$ and $R^4$ are as defined in claim 1, which comprises the acid hydrolysis of a compound of the formula (VI).

7. A process for preparing a compound of the formula (XVIII):

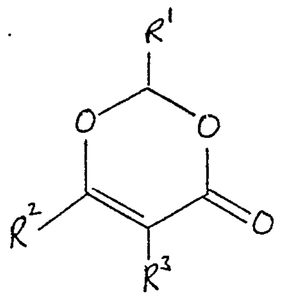

wherein $R^1$, $R^2$ and $R^3$ are as defined in claim 1 with the proviso that $R^3$ is not hydrogen or methyl: which comprises

(i) eliminating the elements $R^{41}$ and hydrogen from a compound of the formula (XIX):

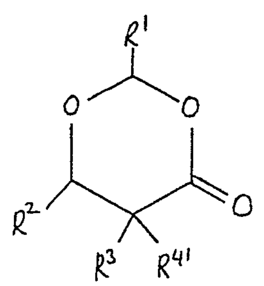

wherein $R^1$, $R^2$ and $R^3$ are as defined in claim 1 and $R^{41}$ is a leaving group; or

(ii) dehydrogenating a compound of the formula (VI), as defined in claim 1, wherein $R^4$ is hydrogen; or

(III) to form a compound of the formula (XVIII) wherein $R^3$ is hydrogen, catalytically hydrogenating a compound of the formula (XX):

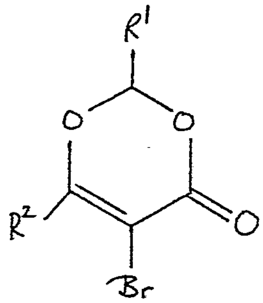

(XX)

wherein $R^1$ and $R^2$ are as hereinbefore defined.